# EUROPEAN PATENT APPLICATION

(11) **EP 3 456 354 A1**
(43) Date of publication of application: **20.03.2019**
(21) Application number: 17905913.4
(22) Date of filing: 20.07.2017
(51) Int. Cl.: A61K 45/00, A61K 31/155, A61K 31/375, A61K 33/08, A61K 33/10, A61P 35/00

(54) **NEW ANTI-MALIGNANT TUMOR AGENT BASED ON SPECIFICITY OF CANCER CELL METABOLISM**

(71) Applicant: Delta-Fly Pharma, Inc., Tokushima-shi Tokushima 771-0116 (JP)
(72) Inventor: WADA Hiromi, Kyoto-shi Kyoto 606-8236 (JP); HAMAGUCHI Reo, Tokyo 107-0052 (JP)
(74) Representative: Hoffmann Eitle
(86) International application number: PCT/JP2017/026372
(87) International publication number: WO 2019/016928

(57) **Abstract**

An object of the present invention is to provide a method for treatment or prevention of cancer, which not only exerts a great effect at low cost but also has few side effects.

A combination drug containing a urinary alkalinization agent and vitamin C or a combination drug containing a urinary alkalinization agent, vitamin C and metformin for use in treatment or remission of cancer.

## Description

### [Technical Field]

The present invention relates to combination use of a urinary alkalinization agent and vitamin C for treatment or remission of cancer.

The present invention also relates to combination use of a urinary alkalinization agent, vitamin C and metformin for treatment or remission of cancer.

### [Background Art]

Recently, it has been confirmed and reported that a cancer immunotherapy using an immune checkpoint inhibitor (trade name: Opdivo (registered trade mark)) using an anti-human PD-1 monoclonal antibody (Nibormab) as an active ingredient is highly effective for treating various cancers, and attracts attention as a novel cancer therapy next to conventional therapies using cytocidal anti-cancer agents and cancer molecular target drugs.

However, the drug cost of Opdivo is extremely high (drug cost per person per year exceeds 20 million yen) and thus puts a heavy burden on patients and the medical insurance systems of country/local governmental units. The medical cost will further increase in the future with rapid aging and medical advances.

Because of this, development of an inexpensive and highly effective method for treatment or prevention of cancer has been desired.

Usually, the intracellular pH (hereinafter referred to as "pHi") value of normal cells is about 6.9 to 7.2 and the extracellular pH (hereinafter referred to as "pHe") value thereof is about 7.3 to 7.4. The pHe of the normal cells is more alkaline than pHi thereof. In contrast, the pHi of cancer cells is about 7.1 to 7.6 and the pHe thereof is 6.2 to 6.9. The pHe of cancer cells is more acidic than pHi thereof. More specifically, the pH gradient between pHi and pHe of cancer cells is reversal compared to that of normal cells.

In cancer cells, compared to normal cells, the glycolysis system is accelerated and productions of lactic acid and protons (hydrogen ions) increase. The lactic acid produced is actively discharged outside the cells by means of a monocarboxylic acid transporter (MCT); whereas protons are actively discharged by means of Na⁺/H⁺ exchanger 1 (NHE-1), Na⁺-dependent HCO₃⁻/Cl⁻ exchanger and H⁺/lactate co-transporter. As a result, the pHe of cancer cells becomes acidic compared with the pHi thereof.

In the cells having a high pHi and highly activated NHE-1, it is reported that malignancy of cells, cell growth, cancer-gene expression, growth-factor activation, hyperactivation of the glycolysis system, DNA-synthesis promotion, promotion of cell-cycling, reduction of apoptosis induction, cell migration, angiogenesis, cancer metastasis and drug resistance can be enhanced, based on a basic research (Non Patent Literature 1).

In cancer cells, NHE-1 is activated and formation of pseudopodia and assembling of lysosomes containing a proteolytic enzyme on pseudopodium tips are accelerated. If NHE-1 is further activated, cancer cells are transfigured like ameba and secretion of the proteolytic enzyme from localized lysosomes is accelerated to facilitate invasion of the cancer cells outside the tissue. In this mechanism, growth of cancer cells is encouraged, as reported in a basic research (Non Patent Literature 2).

If the pHi of human lung cancer cells rises from 7.0 to 7.4, drug resistance to an anti-cancer agent significantly increases. A basic research reports that the resistance to doxorubicin hydrochloride (trade name: Adriamycin (registered trade mark)) increased 2,000 times (Non Patent Literature 3).

Non Patent Literature 4 reports, based on the results of basic researches, that if a plasma concentration of sodium hydrogen carbonate is slowly raised, the pHe of cancer cells can be changed from an acid range to an alkaline range, with the result that growth and invasion of cancer cells may be possibly suppressed.

In more than half of human colorectal cancers, a mutation is found in KRAS gene and BRAF gene. It is known that colorectal cancers having the mutation are intractable to a treatment using a cancer molecular target drug (for example, cetuximab (trade name: Erbitux (registered trade mark))) acting on the epithelium growth factor receptor (EGFR). Whereas, it is reported that colorectal cancer cells having a mutation in KRAS gene and BRAF gene are killed if the cells are in contact with high-concentration vitamin C, in a basic research (Non Patent Literature 5).

It is also reported that a biguanide diabetes drug, metformin, may suppress growth of cancer cells and may be possibly used in combination with other therapies such as a postoperative adjuvant therapy and a chemoprevention therapy (Non Patent Literature 6).

Further, it is reported that when risk of development of pancreatic cancer was compared between diabetic patients taking metformin and those taking no metformin, the risk of development of pancreatic cancer of the diabetic patients taking metformin was reduced by 62 %, (Non Patent Literature 7).

However, up to present, there is no clinical evidence suggesting that a treatment using an alkaline agent such as sodium hydrogen carbonate and vitamin C in combination and a treatment using these and metformin in combination are effective for treatment and/or remission of cancer patients.

### [Citation List]

### [Non Patent Literature]

[Non Patent Literature 1] S. Harguindey, et al., Biochimica et Biophysica Acta 1756 (2005): 1-24
[Non Patent Literature 2] Rosa A. Cardone, et al., Nature Reviews, Cancer 5 (2005): 786-795
[Non Patent Literature 3] H. G. Keizer, et al., J. Natl. Cancer Inst. 81 (1989): 706-709
[Non Patent Literature 4] Ariosto S. Silva, et al., Cancer Research 69 (2009): 2677-2684
[Non Patent Literature 5] Jihye Yun et al., Science 350 (2015): 1391-1396
[Non Patent Literature 6] Shumei Meng, J Endocrinol Diabetes Obes 2 (2014): 1030
[Non Patent Literature 7] Sai-Ching J. Yeung, Gastroenterology 137 (2009): 482-488

### [Summary of Invention]

### [Technical Problem]

With rapid aging and medical advances, medical cost increases more than ever as mentioned above and put a heavy burden on patients and the medical insurance systems of country/local governmental units.

Nowadays, various anti-cancer agents have been developed and clinically used; however, many anti-cancer agents are associated with serious side effects. Such a treatment mean has serious and intolerable side effects on patients, in most cases.

In the circumstances, an object of the present invention is to provide a method for treatment and/or remission of cancer, which not only exerts a great effect at low cost but also has few side effects.

### [Solution to Problem]

The present inventors conducted intensive studies with a view to attaining the aforementioned object. As a result, we found that administration of a urinary alkalinization agent and vitamin C in combination as well as administration of a urinary alkalinization agent, vitamin C and metformin in combination are useful in treatment and/or remission of cancer patients. We also found that in addition to a combination of a urinary alkalinization agent and vitamin C or a combination of a urinary alkalinization agent, vitamin C and metformin, administration of an anti-cancer agent (in a dose much lower than a prescribed amount) in combination therewith is effective for treatment and/or remission of cancer patients. The present invention was based on these findings.

More specifically, the present invention includes the following inventions.
[1] A combination drug containing a urinary alkalinization agent and vitamin C for use in treatment or remission of cancer.
[2] The drug according to [1], wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.
[3] The drug according to [2], wherein the sodium hydrogen carbonate is orally administered in a dosage of 3 to 15 g/day.
[4] The drug according to any one of [1] to [3], wherein the vitamin C is parenterally administered in a dosage of 25 to 50 g/day.
[5] The drug according to any one of [1] to [4], further containing metformin.
[6] The drug according to [5], wherein the metformin is orally administered in a dosage of 250 mg to 500 mg/day.
[7] The drug according to any one of [1] to [6], further comprising an anti-cancer agent.
[8] The drug according to any one of [1] to [7], wherein the cancer is progressive cancer and/or last-stage cancer.
[9] A pharmaceutical composition containing a urinary alkalinization agent to be administered in combination with vitamin C, for use in treatment or remission of cancer.
[10] A pharmaceutical composition containing vitamin C to be administered in combination with a urinary alkalinization agent, for use in treatment or remission of cancer.
[11] A pharmaceutical composition comprising metformin to be administered in combination with a urinary alkalinization agent and vitamin C, for use in treatment or remission of cancer.
[12] The pharmaceutical composition according to any one of [9] to [11], wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.
[13] The pharmaceutical composition according to [12], wherein the sodium hydrogen carbonate is orally administered in a dosage of 3 to 15 g/day.
[14] The pharmaceutical composition according to any one of [9] to [13], wherein the vitamin C is parenterally administered in a dosage of 25 to 50 g/day.
[15] The pharmaceutical composition according to [11], wherein the metformin is orally administered in a dosage of 250 mg to 500 mg/day.
[16] The pharmaceutical composition according to any one of [9] to [15], to be administered in combination with an anti-cancer agent.
[17] The pharmaceutical composition according to any one of [9] to [16], wherein the cancer is progressive cancer and/or last-stage cancer.
[18] A urinary alkalinization agent for use in a method for treatment or remission of cancer together with vitamin C.
[19] The urinary alkalinization agent according to [18], wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.
[20] The urinary alkalinization agent according to [19], wherein the sodium hydrogen carbonate is orally administered in a dosage of 3 to 15 g/day.
[21] The urinary alkalinization agent according to any one of [18] to [20], wherein the vitamin C is parenterally administered in a dosage of 25 to 50 g/day.
[22] The urinary alkalinization agent according to any one of [18] to [21], for use in the method further together with the anti-cancer agent.
[23] The urinary alkalinization agent according to any one of [18] to [22], wherein the cancer is progressive cancer and/or last-stage cancer.
[24] Vitamin C for use in a method for treatment or remission of cancer together with a urinary alkalinization agent.
[25] The vitamin C according to [24], to be parenterally administered in a dosage of 25 to 50 g/day.
[26] The vitamin C according to [24] or [25], wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.
[27] The vitamin C according to [26], wherein the sodium hydrogen carbonate is orally administered in a dosage of 3 to 15 g/day.
[28] The vitamin C according to any one of [24] to [27], for use in the method further together with an anti-cancer agent.
[29] The vitamin C according to any one of [24] to [28], wherein the cancer is progressive cancer and/or last-stage cancer.
[30] Metformin to be used in a method for treatment or remission of cancer together with a urinary alkalinization agent and vitamin C.
[31] The metformin according to [30] to be orally administered in a dosage of 250 mg to 500 mg/day.
[32] The metformin according to [30] or [31], wherein the vitamin C is parenterally administered in a dosage of 25 to 50 g/day.
[33] The metformin according to any one of [30] to [32], wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.
[34] The metformin according to [33], wherein the sodium hydrogen carbonate is orally administered in a dosage of 3 to 15 g/day.
[35] The metformin according to any one of [30] to [34], for use in the method further with an anti-cancer agent.
[36] The metformin according to any one of [30] to [35], wherein the cancer is progressive cancer and/or last-stage cancer.
[37] A method for treatment or remission of cancer, comprising administering a urinary alkalinization agent and vitamin C to a cancer patient.
[38] The method according to [37], wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.
[39] The method according to [38], wherein the sodium hydrogen carbonate is orally administered in a dosage of 3 to 15 g/day.
[40] The method according to any one of [37] to [39], wherein the vitamin C is parenterally administered in a dosage of 25 to 50 g/day.
[41] The method according to any one of [37] to [40], comprising further administering metformin.
[42] The method according to [41], wherein the metformin is administered in a dosage of 250 mg to 500 mg/day.
[43] The method according to any one of [37] to [42], comprising further administering an anti-cancer agent.
[44] The method according to any one of [37] to [43], wherein the cancer is progressive cancer and/or last-stage cancer.
[45] Use of a urinary alkalinization agent and/or vitamin C in producing a medicament for use in a method for treatment or remission of cancer, comprising administering a urinary alkalinization agent and vitamin C to a cancer patient.
[46] The use according to [45], comprising further administering metformin in the method.
[47] Use of metformin for producing a medicament for use in a method for treatment or remission of cancer, comprising administering a urinary alkalinization agent, vitamin C and metformin to a cancer patient.
[48] The use according to any one of [45] to [47], wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.
[49] The use according to [48], wherein the sodium hydrogen carbonate is orally administered in a dosage of 3 to 15 g/day.
[50] The use according to any one of [45] to [49], wherein the vitamin C is parenterally administered in a dosage of 25 to 50 g/day.
[51] The use according to [46] or [47], wherein the metformin is orally administered in a dosage of 250 mg to 500 mg/day.
[52] The use according to any one of [45] to [51], comprising further administering an anti-cancer agent in the method.
[53] The use according to any one of [45] to [52], wherein the cancer is progressive cancer and/or last-stage cancer.

### [Advantageous Effects of Invention]

According to the present invention, it is possible to provide a method for treatment and/or remission of cancer, which not only exerts a great effect at low cost but also has few side effects.

### [Brief Description of Drawings]

Figure 1 is a graph showing the measurement results of the tumor marker (CA19-9) levels (U/ml) in the serum of a patient with unresectable progressive pancreatic cancer before and after administration of sodium hydrogen carbonate and vitamin C.
Figure 2 is a graph showing the measurement results of the tumor marker (CA19-9) levels (U/ml) in the serum of a patient with recurrent pancreatic cancer (pancreatic body cancer) before and after administration of sodium hydrogen carbonate, vitamin C, Gemzar (registered trade mark) and metformin.
Figure 3-1 is a graph showing the measurement results of the tumor marker (CEA) level (ng/ml) in the serum and urine pH value of a patient with non-small-cell lung cancer (adenocarcinoma) before and after administration of sodium hydrogen carbonate, vitamin C, Alimta (registered trade mark) and Avastin (registered trade mark).
Figure 3-2 showing CT images of the thoracic cavity of a patient with non-small-cell lung cancer (adenocarcinoma) before and after administration of sodium hydrogen carbonate, vitamin C, Alimta (registered trade mark) and Avastin (registered trade mark). The white arrows show metastatic cancers scattered in the thoracic cavity.
Figure 4-1 is a graph showing the measurement results of urine pH value of a patient with malignant pleural mesothelioma before and after administration of sodium hydrogen carbonate, vitamin C and Alimta (registered trade mark).
Figure 4-2 is chest x-ray photos of a patient with malignant pleural mesothelioma before and after administration of sodium hydrogen carbonate, vitamin C and Alimta (registered trade mark). The white arrows indicate malignant pleural mesotheliomas scattered in the right lung.
Figure 5-1 is a graph showing the measurement results of urine pH value of a patient with pancreatic cancer (pancreatic body cancer) before and after administration of sodium hydrogen carbonate, vitamin C and Gemzar (registered trade mark).
Figure 5-2 shows CT images of the liver and pancreatic body of a patient with pancreatic cancer (pancreatic body cancer) before and after administration of sodium hydrogen carbonate, vitamin C and Gemzar (registered trade mark). The white arrows indicate pancreatic cancer; whereas the black arrows indicate metastatic cancers scattered in the liver.
Figure 6-1 is a graph showing measurement results of urine pH value, HbA1c value, and CRP value and N/L ratio of a patient with pancreatic cancer (pancreatic body cancer) before and after administration of sodium hydrogen carbonate, vitamin C, Gemzar (registered trade mark) and metformin.
Figure 6-2 is a graph showing measurement results of tumor marker (CA19-9) level (U/ml) and tumor marker (CEA) level (ng/ml) in the serum of a patient with pancreatic cancer (pancreatic body cancer) before and after administration of sodium hydrogen carbonate, vitamin C and Gemzar (registered trade mark).
Figure 6-3 shows CT images of the liver and pancreatic body of a patient with pancreatic cancer (pancreatic body cancer) before and after administration of sodium hydrogen carbonate, vitamin C and Gemzar (registered trade mark)
Figure 7 is a graph showing the life extension rate of patients with advanced pancreatic cancer who was treated by the method of the present invention for two years after initiation of treatment by the method of the present invention.

### [Description of Embodiments]

The present invention relates to a method for treatment or remission of cancer, comprising administering, to a cancer patient, a combination of a urinary alkalinization agent and vitamin C or a combination of a urinary alkalinization agent, vitamin C and metformin, and if necessary, further administering an anti-cancer agent; and relates to a drug or a combination drug used in the method.

In the present invention, the "urinary alkalinization agent" refers to an agent having an action to alkalinize extracellular pH (pHe) of cancer cells of the subject to which the agent was administered and/or acidize pHi thereof. Whether the agent has the action or not can be determined based on the pH value of urine of the subject to which the agent was administered. In the subject to which the agent was administered, if pH value of the urine slightly shifts toward an alkaline pH range and/or is maintained at an alkaline pH, it can be determined that the compound administered has said action. The determination is made by collecting urine immediately after administration of the agent or after continuous administration period (for example, 1 month, 2 months, 3 months, 4 months, 5 months, or 6 month after administration) and measuring the pH value of the urine collected. In the present invention, "urine alkalinization" refers to the state of urine exhibiting a pH value of beyond 7, preferably 7.5 or more and more preferably 8 or more.

In the present invention, as an available "urinary alkalinization agent", a compound generating a bicarbonate ion (HCO₃⁻) *in vivo* and a compound having an antacid action, are mentioned. Examples of the compound having such an action include, but are not limited to, a carbonate, a hydrogen carbonate, a citrate, an acetate, a lactate, an oxide, a hydroxide and a silicate. Examples of the salts include alkali metal salts (sodium salt, potassium salt, etc.), alkaline earth metal salts (calcium salt, etc.), magnesium salts and aluminum salts.

In the present invention, specific examples of the available "urinary alkalinization agent" include sodium hydrogen carbonate (baking soda), sodium carbonate, calcium carbonate, sodium citrate, potassium citrate, sodium acetate, sodium lactate, potassium lactate, magnesium oxide, magnesium hydroxide, aluminum hydroxide and aluminum silicate. Preferably, sodium hydrogen carbonate is mentioned.

The "urinary alkalinization agent" of the present invention may include a single or a plurality of compounds selected from the aforementioned examples.

The "urinary alkalinization agent" of the present invention may be industrially synthesized in accordance with a method conventionally known in the art; alternatively, a commercially available "urinary alkalinization agent" for foods or pharmaceutical products may be used.

In the present invention, "vitamin C" refers to L-ascorbic acid (hereinafter referred to as "ascorbic acid"), a salt thereof, an ester thereof and a derivative thereof. Specific examples thereof include ascorbic acid, sodium ascorbate, potassium ascorbate, ascorbic acid monostearate, ascorbic acid monopalmitate and ascorbic acid monooleate. Preferably, ascorbic acid is mentioned.

In the present invention, "vitamin C" may be industrially synthesized in accordance with a method conventionally known in the art; alternatively, commercially available one for foods or pharmaceutical products may be used.

In the present invention, "metformin" refers to a compound represented by the following formula: 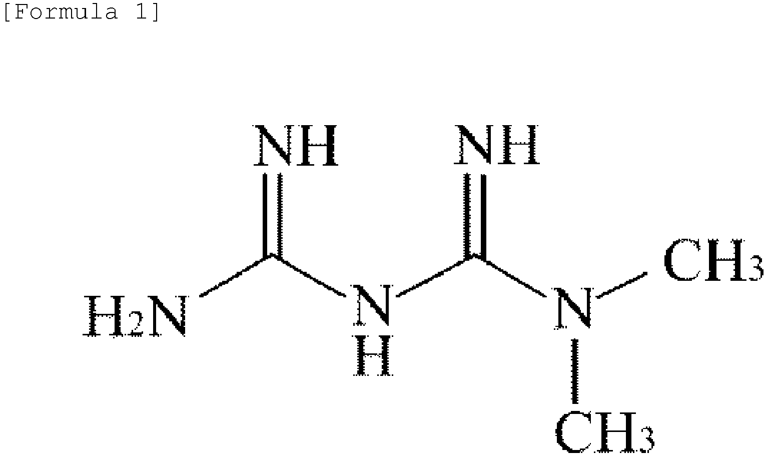 or a salt thereof. The "salt" is not limited as long as it is a pharmacologically acceptable salt; for example, an acid addition salt and a basic addition salt are mentioned. Examples of the "acid addition salt" include (but are not limited to), a hydrochloride, a sulfate, a nitrate, a phosphate, a carbonate and an acetate. Examples of the "basic addition salt" include (but are not limited to), alkali metal salts (sodium salt, potassium salt, etc.), alkaline earth metal salts (calcium salt, etc.), magnesium salts and ammonium salts. "Metformin" in the present invention is preferably a metformin hydrochloride.

In the present invention, "metformin" may be industrially synthesized by a method conventionally known in the art; alternatively commercially available one as a pharmaceutical products having an effect/efficacy for Type 2 diabetes may be used.

In the present invention, examples of the "anti-cancer agent" include existing anti-cancer agents and molecular target drugs for use in a method for treatment or remission of cancer. Examples of such an "anti-cancer agent" include, but are not limited to, tegafur, tegafururacil combination preparation, tegafur-gimeraciloteracil potassium combination preparation (trade name: TS-1 (registered trade mark)), fluorouracil, gemcitabine (trade name: Gemzar (registered trade mark)), enocitabine, carmofur, doxifluridine, cytarabine, cytarabine ocfosphate, mercaptopurine, fludarabine, capecitabine, methotrexate, cladribine, pemetrexed (trade name: Alimta (registered trade mark)), hydroxycarbamide, cyclophosphamide, ThioTEPA, ifosfamide, busulfan, dacarbazine, melphalan, ranimustine, nimustine, temozolomide, carboplatin, cisplatin, oxaliplatin, nedaplatin, doxorubicin, aclarubicin, idarubicin, actinomycin D, daunorubicin, zinostatin stimalamer, bleomycin, mitomycin C, pirarubicin, epirubicin, peplomycin, amrubicin, vinca alkaloid, taxane, topoisomerase inhibitors, sorafenib, erlotinib, axitinib, everolimus, sunitinib, imatinib, lapatinib, rituximab, dasatinib, bortezomib, tamibarotene, gefitinib, ibritumomab, nilotinib, temsirolimus, trastuzumab, panitumumab, tretinoin, gemtuzumab ozogamicin, crizotinib, afatinib, bevacizumab (trade name: Avastin (registered trade mark)) and paclitaxel (trade name: Abraxane (registered trade mark)).

In the present invention, the "cancer" includes solid cancers and blood cancers (for example, leukemia, malignant lymphoma and multiple myeloma), and is preferably a solid cancer. Examples of the solid cancer include all solid cancers (more specifically, epithelium cell cancers and non epithelium cell cancers) except blood cancers. Examples of such a solid cancer include, but are not limited to, brain tumor/glioma, pituitary adenoma, acoustic neurinoma, uveal melanomas, meningioma, pharyngeal cancer, laryngeal cancer, tongue cancer, thyroid cancer, breast cancer, lung cancer, thymoma, thymic carcinoma, mesothelioma, esophagus cancer, stomach cancer, colorectal cancer, hepatocarcinoma, bile duct cancer, pancreatic cancer, renal cellular cancer, bladder cancer, prostate cancer, renal pelvis/ureteral cancer, penile cancer, testes (testicles) tumor, uterine cancer, ovarian cancer, vulvar cancer, skin cancer, malignant melanoma (skin), basal cell cancer, skin cancer prodromal symptom, epidermis cancer, squamous cell cancer, mycosis fungoidesis, malignant bone tumor (osteosarcoma), soft tissue sarcoma, chondrosarcoma, malignant fibrous histiocytoma and, metastatic cancers of these.

Preferably, in the present invention, "cancer" refers to a cancer having a mutation in KRAS gene and/or BRAF gene. The KRAS gene is a gene encoding a low-molecular GTP binding protein (about 21kDa) having a role of transmitting a proliferation signal from EGFR to downstream. In colorectal cancer, a mutation is found in KRAS gene in a proportion of 45 to 50%; about 70% of them are known to be mutations in codon 12; whereas about 30% of them are mutations in codon 13 (Yamazaki K et al.: ESMO 201 0: abst #595P). Also in the present invention, as a mutation in the KRAS gene, a mutation of codon 12 and/or 13 can be used. It is known that if the KRAS gene has a mutation, a therapeutic effect by an anti-EGFR antibody drug is not sufficiently obtained (Amado RG et al.: J Clin Oncol.26 (10): 1626-1634, 2008; Karapetis CS et al.: N Engl J Med. 359 (17): 1757-1765, 2008; Dahabreh IJ et al.: Ann Intern Med. 154 (1): 37-49, 2011). The BRAF gene is a gene encoding serine/threonine kinase, which constitutes the Ras-Raf-MAPK pathway. In colorectal cancer, a mutation in the KRAS gene is found in a proportion of 5 to 10%. About 90% of them are known to be mutations in codon 600 (Clancy C et al.: Colorectal Dis. 15 (12): e711-718, 2013; Smith CG et al.: Clin Cancer Res. 19 (15): 4104-4113, 2013). Also in the present invention, mutation in codon 600 can be used as a mutation in BRAF gene. It is known that if the BRAF gene has a mutation, a therapeutic effect by an anti-EGFR antibody drug cannot be sufficiently obtained. Mutations of individual genes can be detected by a genetic test method (for example, direct sequencing method, Allele-Specific PCR assay) conventionally known in the art.

Preferably, in the present invention, examples of "cancer" include refractory cancer such as progressive cancer (stage II to IV) and/or last-stage cancer (stage IV). Examples of the cancer that can be treated or brought into remission in the present invention include, but are not limited to, progressive and/or last-stage pancreatic cancer, lung cancer and pleural mesothelioma.

In the present invention, a urinary alkalinization agent and vitamin C are administered in combination to a cancer patient.

In the present invention, "administered in combination" includes not only a case where individual components are simultaneously administered but also a case where individual components are sequentially administered at predetermined intervals during a treatment period (combination therapy). The administration routes and administration means of individual components to be administered in combination may be the same or different.

The dosage amount and administration route of a urinary alkalinization agent may vary depending on factors such as the type and severity of cancer, and the age, body weight and state of a patient; however, it is satisfactory if a sufficient amount of the agent, which can change the pH value of urine of the patient (to which the urinary alkalinization agent was administered) toward an alkaline pH and/or maintain the pH value within the alkaline range, can be administered through any administration route (oral administration or parenteral administration).

For example, if a urinary alkalinization agent is sodium hydrogen carbonate, the dosage amount, which is selected from 3 to 15 g and preferably 3 to 9 g, is divided into 1 to 5 doses (for example, 2 or 3 doses) per day and orally administered every day, every other day or at intervals of several days. For example, sodium hydrogen carbonate of 3 g, 6 g or 9 g per day is divided into 1 to 2 doses and orally administered every day.

The dosage amount and administration route of vitamin C vary depending on factors such as the type and severity of cancer, and the age, body weight and state of a patient; however, any dosage amount and administration route can be employed. For example, the dosage amount of vitamin C selected from 10 to 50 g/day and preferably 25 to 50 g/day is parenterally administered once a week, 2 to 3 days per month, once every other week or once a month. As the "parenteral administration", e.g., intravenous injection, subcutaneous injection, intradermal injection and intramuscular injection can be mentioned. For example, vitamin C can be intravenously injected in a dosage amount of 25 g/day, once a week, 2 to 3 days per month, once every other week or once a month. Alternatively, vitamin C can be orally administered in a dosage in such a way that the amount of vitamin C in the veins corresponds to the amount of vitamin C in the veins when it is parenterally administered.

In the present invention, in addition to a urinary alkalinization agent and vitamin C, metformin can be further administered in combination therewith. The "administered in combination" herein is the same as defined in the above.

The dosage amount and administration route of metformin vary depending on factors such as the type and severity of cancer, and the age, body weight and state of a patient; however, any dosage amount and administration route can be employed. For example, the dosage amount of metformin, which is selected from 100 mg to 500 mg/day, preferably, 250 mg to 500 mg/day, is divided into 1 to several doses (2 or 3 doses) per day and orally administered every day, every other day or at intervals of several days. For example, 250 mg of metformin is divided into 2 or 3 doses per day and can be orally administered every day.

Note that, the cancer patient which receives administration in combination with metformin may or may not have diabetes (Type 2 diabetes) and the cancer patient is not limited to a diabetic patient.

The urinary alkalinization agent, vitamin C and metformin that are administered in combination may be provided in the form of discrete drugs (or pharmaceutical compositions) or in the form of a combination drug.

In the case where these components are provided as discrete drugs (or pharmaceutical compositions), since these drugs (or pharmaceutical compositions) are designed to be used in combination as above, combination use thereof can be instructed in a package insert, more specifically, in the "efficacy-effect" section or "dosage and administration" section. For example, in the case of a drug (or a pharmaceutical composition) containing a urinary alkalinization agent, the instruction that the drug should be "used in combination with vitamin C" or "used in combination with vitamin C and metformin" for treatment or remission of cancer, can be provided in its package insert. In the case of a drug (or a pharmaceutical composition) containing vitamin C, the instruction that the drug should be "used in combination with a urinary alkalinization agent" or "used in combination with a urinary alkalinization agent and metformin", for treatment or remission of cancer, can be provided in its package insert. In the case of a drug (or a pharmaceutical composition) containing metformin, the instruction that the drug should be "used in combination with a urinary alkalinization agent and vitamin C", for treatment or remission of cancer, can be provided in its package insert.

A combination drug may be provided in the form of a combination preparation containing individual components in a single composition or in the form (i.e., a kit) where individual components are separately prepared, and manufactured, packaged, and distributed in a single package suitable for combined administration.

The above drug (or a pharmaceutical composition) and combination drug may further contain, e.g., an excipient, a binder, a disintegrant and a lubricant, which are usually used for producing drugs, in addition to the aforementioned components and can be produced in a dosage form suitable for an intended administration route.

Examples of the excipient include sugar (monosaccharide, disaccharide, polysaccharide such as cyclodextrin and alginic acid), a metal salt, kaolin, silicic acid, polyethylene glycol and a mixture of these.

Examples of the binder include a single syrup, a glucose solution, a starch solution, a gelatin solution, polyvinyl alcohol, polyvinyl ether, polyvinylpyrrolidone, carboxymethyl cellulose, shellac, methylcellulose, ethylcellulose and a mixture of these.

Examples of the disintegrant include dry starch, sodium alginate, powdered agar, laminarin powder, sodium hydrogen carbonate, calcium carbonate, a polyoxyethylene sorbitan fatty acid ester, sodium lauryl sulfate, monoglyceride stearate, starch, lactose and a mixture of these.

Examples of the lubricant include purified talc, stearate, borax, polyethylene glycol and a mixture of these.

If necessary, further a diluent, a stabilizer, a tonicity agent, a pH modifier, a buffer, a solubilizing agent, a suspending agent, a colorant, a corrigent, flavoring agent, a coating agent, a preservative, an antiseptic agent and an antioxidant, which are usually used in producing drugs, can be appropriately contained.

As the dosage form suitable for oral administration, for example, tablets, pills, capsules, granules, powders, syrups and suspensions are mentioned. If the dosage form is solid form, if necessary, coating may be applied to the dosage form (for example, sugar-coated tablets, gelatin coated tablets, enteric coated tablets).

As the dosage form suitable for parenteral administration, e.g., injections and intravenous fluids are mentioned. These dosage forms may be provided in a lyophilized and preservable state; when used, they are each dissolved in a buffer containing water or physiological saline to prepare a solution having an appropriate concentration and then put in use.

In the present invention, in addition to a combination of a urinary alkalinization agent and vitamin C or a combination of a urinary alkalinization agent, vitamin C and metformin, an anti-cancer agent is further administered in combination therewith. The "administered in combination" herein is the same as defined above.

As the anti-cancer agent to be used in the present invention, one or a plurality of anti-cancer agents are appropriately selected depending on factors such as the type and severity of cancer, and the age, body weight and state of a patient.

The anti-cancer agent can be used in dosage and administration sufficient to maintain immune function of a patient to which the anti-cancer agent was administered or so as not to remarkably reduce (at a rate of, for example, 40% or more, 50% or more, 60% or more, 70% or more, or more) the immune function. Variation of patient's immune function can be determined based on one or a plurality of values selected from the count numbers of neutrophils, granulocytes, monocytes, lymphocytes and platelets in the peripheral blood.

The anti-cancer agent to be used in combination with a urinary alkalinization agent and vitamin C or a urinary alkalinization agent, vitamin C and metformin can be administered in a dosage which is 90%, 80%, 70%, 60%, 50%, 40% or less as low as the dosage of the case where the anti-cancer agent is used alone, and/or, in a reduced administration period and/or following drug regimen having extended drug holidays. Owing to this, occurrence of a side effect(s) (e.g., marrow suppression, hemolytic anemia, disseminated intravascular coagulation syndrome, fulminant hepatitis, dehydration symptoms, enteritis, interstitial pneumonia, stomatitis, gastrointestinal ulcer, gastrointestinal bleeding, gastrointestinal perforation, acute renal failure, skin mucosal ocular syndrome, toxic epidermal necrosis, psychoneuropathy, acute pancreatitis, rhabdomyolysis and olfactory anesthesia, but examples are not limited to these) caused by administration of the anti-cancer agent can be suppressed or delayed.

The anti-cancer agent to be administered in combination therewith may be provided in the form of a discrete drug (or a pharmaceutical composition) from a urinary alkalinization agent, vitamin C and metformin or in the form of a combination drug such as a combination preparation or a kit preparation, with other components.

In the case where an anti-cancer agent is provided as a discrete drug (or a pharmaceutical composition), instruction that the agent should be used for combined administration as mentioned above can be provided in a package insert of the anti-cancer agent, more specifically, in the "efficacy-effect" section or "dosage and administration" section. More specifically, the instruction that the anti-cancer agent should be "used in combination with a urinary alkalinization agent and vitamin C" or "used in combination with urinary alkalinization agent, vitamin C and metformin" for treatment or remission of cancer, can be provided in the package insert of the anti-cancer agent.

According to the present invention, it is possible to inhibit or suppress one or a plurality of mechanisms in connection with malignant alteration, growth and metastasis of cancer, such as malignant alteration of cells, cell growth, cancer-gene expression, growth-factor activation, hyperactivation of the glycolysis system, DNA-synthesis promotion, cell-cycle hyperactivation, decrease of apoptosis induction, cell migration, angiogenesis, cancer metastasis and drug resistance, in the cancer patient treated with the combined administration. Owing to this, cancer can be calm down and treated or brought into remission. Particularly, the effect of the invention is markedly observed in refractory (progressive and/or last-stage) cancers and a survival rate thereof can be greatly enhanced.

The urinary alkalinization agent and vitamin C to be used in the present invention are relatively inexpensively produced and sold. In addition, metformin and an anti-cancer agent to be administered in combination are used in a lower amount than that used in conventional treatment methods. Because of this, the present invention can reduce economic burden on patients and the medical insurance systems of country/local governmental units.

### [Examples]

Now, the present invention will be more specifically described by way of Examples, below; however, the present invention is not limited to these.

### [Example 1]

Patient (male, 78 years old) with unresectable progressive pancreatic cancer (Stage IVb)

The patient (male, 78 years old) was definitely diagnosed as unresectable progressive pancreatic cancer (Stage IVb) accompanied by liver metastasis and abdominal lymph node metastasis on February 2016.

From March 2016, edible sodium hydrogen carbonate (baking soda) was taken (3 g per time) twice per day and vitamin C (25 g/day) was intravenously administered once per month.

As a result, urine (pH) was alkalinized (pH = 8.0) from the end of June 2016 and thereafter maintained within an alkaline range.

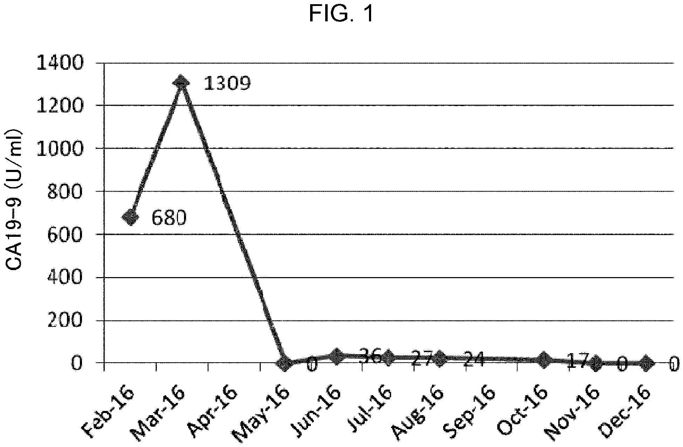

The level of a tumor marker (CA19-9) in the serum was 1309 (U/ml) (March 2016) before sodium hydrogen carbonate and vitamin C were administered; whereas the level became 0 (U/ml) two months after the administration (May 2016) and thereafter was maintained low (Figure 1).

Further, a primary lesion in the pancreas shrank from 3 cm to 1 cm and a metastatic lesion in the liver also shrank.

Since a dramatic therapeutic effect was exerted by administration of sodium hydrogen carbonate and vitamin C, an anti-cancer agent was not used.

### [Example 2]

Patient (male, 67 years old) with recurrent pancreatic cancer (pancreatic body cancer) (Stage IVa)

The patient (male, 67 years old) underwent pancreatosplenectomy, D2 dissection and surgical resection of the transverse mesocolon on the end of June 2016. A few months later, the patient was diagnosed as recurrent pancreatic cancer (pancreatic body cancer) (Stage IVa, cT4N1M0 (Classification of Pancreatic Carcinoma)). The patient also had diabetes.

From the middle of August 2016, oral administration of TS-1 (registered trade mark) (Taiho Pharmaceutical Co., Ltd.) was started; however, the administration was immediately terminated due to a side effect (eruption).

In the middle of October 2016, intravenous administration of Gemzar (registered trade mark) (Eli Lilly Japan, K.K.) in a reduced dosage amount (800 mg/m²) corresponding to 80% of a prescribed amount, was started every other week. However, in the middle of December 2016, the patient was suspected to have peritoneal dissemination based on MRI inspection and the level of a tumor marker (CA19-9) significantly increased.

Then, from the middle of February 2017, in addition of the intravenous administration of Gemzar (800 mg/m²) every other week, sodium hydrogen carbonate (3 g/day) was taken every day and vitamin C (25 g/day) was intravenously administered once a week. In addition, metformin hydrochloride was administered every day in a reduced amount (250 mg/day) corresponding to 50% of a prescribed amount.

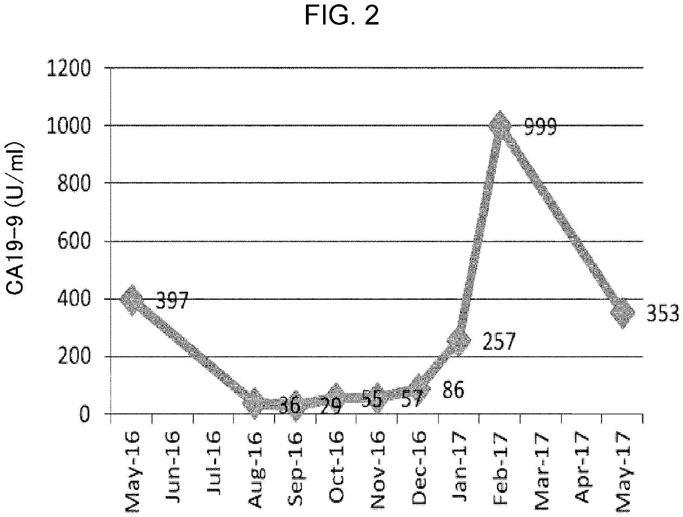

As a result, the level (U/ml) of a tumor marker (CA19-9) dramatically decreased after peaked on February 2017 (Figure 2). In addition, the urine (pH) of the patient was maintained within an alkaline range and HbA1c value in the blood was maintained within a normal range. Whereas, a side effect was not observed.

### [Example 3]

Patient (male, 80 years old) with non-small-cell lung cancer (adenocarcinoma) (Stage IV)

The patient (male, 80 years old) had non-small-cell lung cancer (adenocarcinoma) (cT2aN3M1b, Stage IV) (no operation history) associated with cancer pleurisy, a 3-cm tumor mass in the upper right lobe, right pleural effusion, multiple pulmonary nodule and lumbar spine L1 metastasis, and was determined that the effect of a molecular targeted drug for regulating EGFR cannot be expected.

In the late of October 2016, radiation (3.5 × 8fr) was applied to the lumbar spine for treating bone metastasis. After that, from the end of December 2016, Alimta (registered trade mark) (Eli Lilly Japan, K.K.) (500 mg/m²) and Avastin (registered trade mark) (Chugai Pharmaceutical Co., Ltd.) (15 mg/kg) were repeatedly administered at intervals of four weeks.

Further, in the late of January, 2017, RANMARK (humanized anti RANKL monoclonal antibody) was administered for ameliorating a bone lesion.

However, no improvement was observed in the lesion and the level (ng/ml) of a tumor marker (CEA) continuously increased.

Then, from February 2017, sodium hydrogen carbonate (9 g/day) was further taken every day and high-dose vitamin C (25 g/day) was intravenously administered once a week.

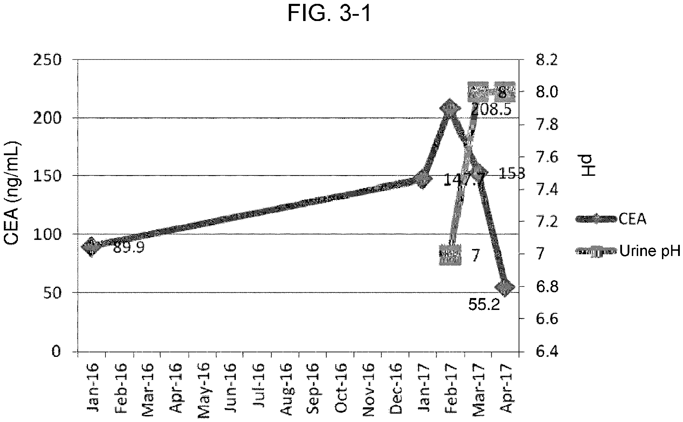

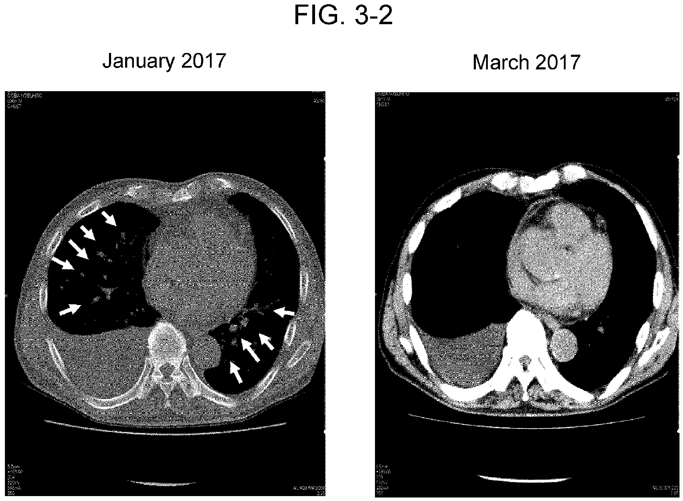

As a result, alkalinization (pH = 8.0) of urine (pH) was immediately observed and the level of a tumor marker (CEA) dramatically decreased (about 210 ng/ml to about 55 ng/ml) (Figure 3-1). As the results of CT inspection, it was confirmed that pleural effusion decreased and metastases scattered in the pleural space disappeared (Figure 3-2, arrow). Side effects were not observed.

### [Example 4]

Patient (male, 67 years old) with malignant pleural mesothelioma

The patient (male, 67 years old) was definitely diagnosed with malignant pleural mesothelioma in the right lung by CT, PET and biopsy, on March 2015. The patient was an architect and likely inhaled asbestos during construction work. A brother of the patient, who was also an architect, was dead of malignant pleural mesothelioma.

The patient received administration in combination with Alimta (registered trade mark) and carboplatin at intervals of three weeks from January 2016.

From January 2017, a single-agent (Alimta) administration (at intervals of 4 weeks) was started in place of the above administration; at the same time, sodium hydrogen carbonate (9 g/day) was taken every day and high-dose (25 g/day, 1 to 2 days per month) vitamin C was intravenously administered.

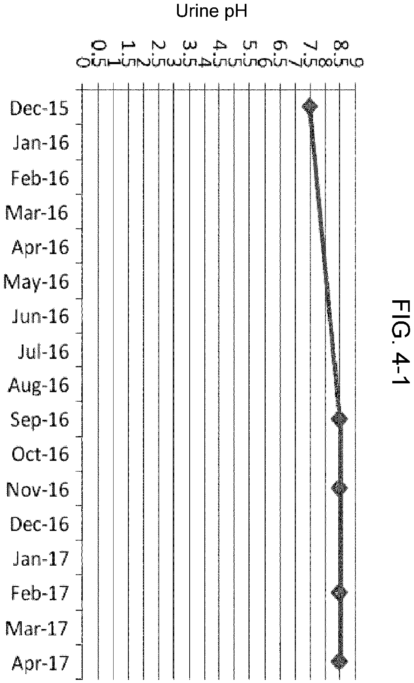

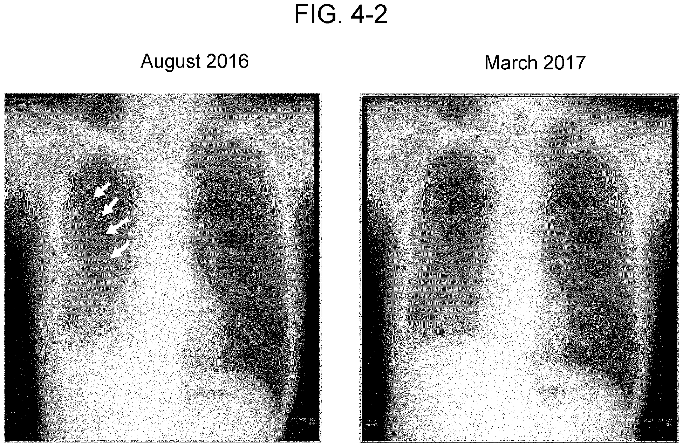

As a result, alkalinization of urine (pH) was maintained (Figure 4-1). An improvement in malignant pleural mesothelioma was observed in a chest X-ray examination (Figure 4-2). Side effects were not observed.

### [Example 5]

Patient (female, 66 years old) with pancreatic cancer (pancreatic body cancer) (Stage IVb)

The patient (female, 66 years old) was definitely diagnosed with pancreatic body cancer (cT4N0M1, Stage IVb) associated with liver metastasis.

From the end of March 2015, the patient received a combination therapy of Gemzar (registered trade mark) (Eli Lilly Japan, K.K.) and Abraxane (registered trade mark) (Taiho Pharmaceutical Co., Ltd.) (one cycle consisting of 4 weeks: 3 times drug administrations (once per week) with one-week drug holydays). However, the combination therapy was stopped because lower limb edema and severe numbness of limbs were observed.

Then, from March 2016, combination use of Abraxane (registered trade mark) was ceased, Gemzar (registered trade mark) was administered in a reduced amount (800 mg/m²) corresponding to 80% of a prescribed amount. Then, the administration regimen was switched to one cycle consisting of 3 weeks: twice drug administrations (once per week) with one-week drug holydays. In addition, sodium hydrogen carbonate (3 g/day, every day, twice) was taken and high-dose vitamin C (25 g/day, 2 to 3 days per month) was intravenously administered.

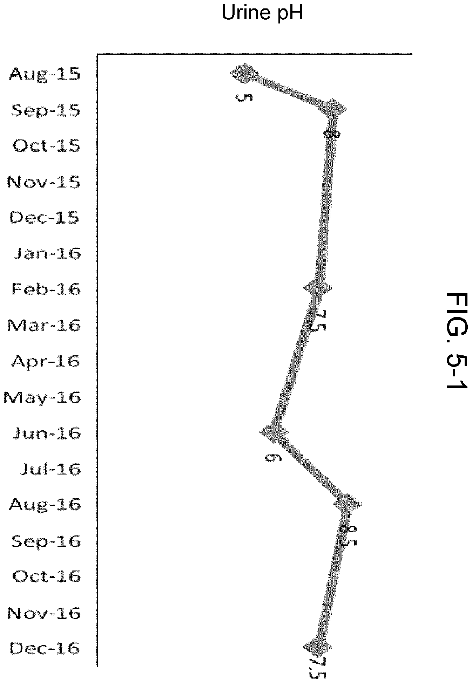

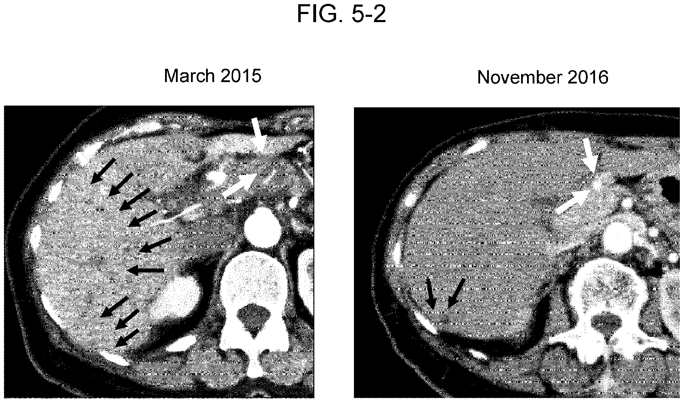

As a result, urine (pH) was alkalinized (pH = 8.0) (Figure 5-1). According to the results of CT inspection, the pancreatic body tumor was reduced in size (Figure 5-2, white arrow) and liver metastasis almost disappeared (Figure 5-2, black arrow). Side effects were not observed.

### [Example 6]

Patient (male, 54 years old) with pancreatic cancer (pancreatic body cancer) (Stage IVb)

The patient (male, 54 years old) underwent test laparotomy in the end of November 2016 and definitely diagnosed with pancreas body cancer (Stage IVb) associated with peritoneal dissemination (dissemination in the Douglas pouch). The patient also had diabetes.

From the early December 2016, sodium hydrogen carbonate (3 g/day, every day, twice) was taken and high-dose vitamin C (25 g/day, 2 to 3 days per month) was intravenously administered. In addition, metformin hydrochloride (tablets) was administered in a reduced amount (250 mg/day) corresponding to 50% of a prescribed amount. Further Gemzar (registered trade mark) (Eli Lilly Japan, K.K.) was intravenously administered every other week in a reduced amount (800 mg/m²) corresponding 80% of a prescribed amount.

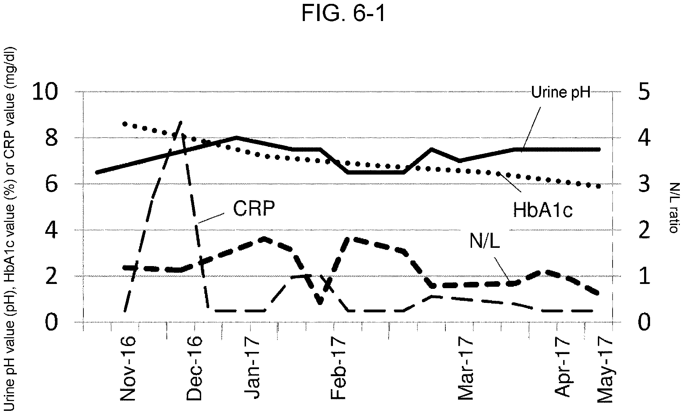

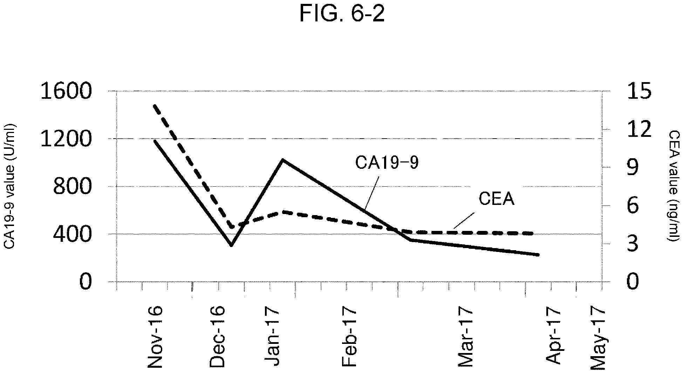

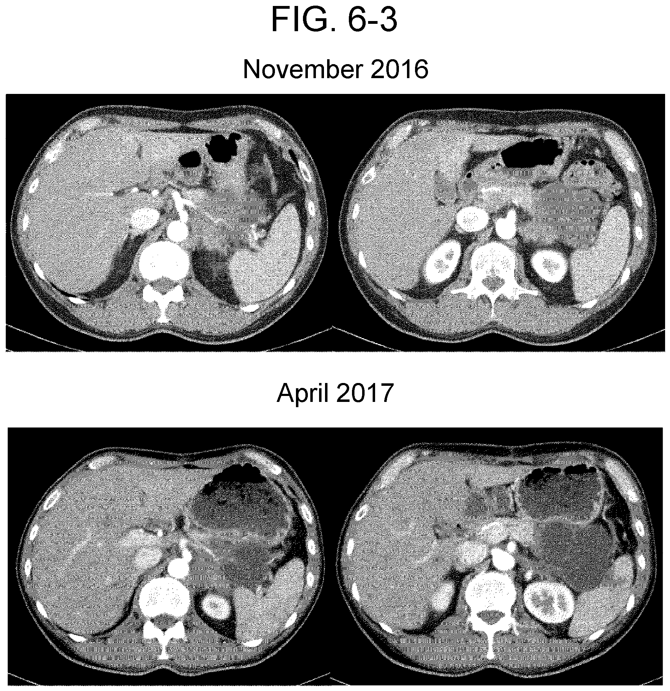

As a result, alkalinization of urine (pH) was maintained. The HbA1c value in the blood decreased to fall within the range of a healthy person (Figure 6-1). Furthermore, a CRP value and a N/L ratio both were maintained low (Figure 6-1). Moreover, the levels of tumor markers, CA19-9 and CEA, both decreased (Figure 6-2). According to the results of CT inspection, distinguishable difference (for example, ascites increase) before and after the administration was not observed. Worsening of peritoneal dissemination was not observed (Figure 6-3). Side effects were not observed.

### [Example 7]

### Life extension effect

According to "Cancer Statistics" published in 2016 by the Foundation for Promotion of Cancer Research, it was reported that the 5-year survival rate of patients with advanced pancreatic cancer (Stage IV) is almost zero (several% or less).

To patients with advanced pancreatic cancer (patients with advanced pancreatic cancer who wished to receive a patient-friendly treatment after definite diagnosis or after postoperative relapse, with or without previous treatment (3 cases: Stage IIIb, 30 cases: Stage IV)), sodium hydrogen carbonate was administered and high-dose vitamin C was intravenously administered. In addition, if necessary, an anti-cancer agent was administered so as not to decrease the immunity of the patients based on the variation of lymphocyte counts as an index. As a result, in 2 of 3 cases of patients with advanced pancreatic cancer (Stage IIIb) and 6 of 30 cases of patients with advanced pancreatic cancer (Stage IV), it was confirmed that the levels of the tumor markers significantly decrease, that tumors are significantly reduced in size by CT inspection, and that the states of patients were notably improved.

Then, sodium hydrogen carbonate was taken and high-dose vitamin C was intravenous administered. In addition, if necessary, an anti-cancer agent was administered so as not to decrease the immunity of the patients based on the variation of lymphocyte counts as an index. In this manner, analysis on persistence (life extension effect) of a therapeutic effect was performed for two years after initiation of this treatment.

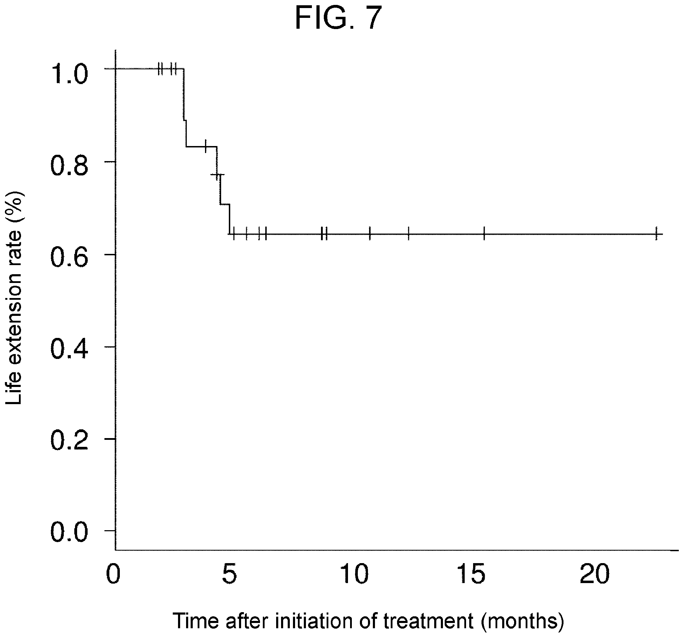

As a result, it was confirmed that 65% of the patients of 33 cases survived (Figure 7). Note that, of the patients with Stage IV pancreatic cancer, who were subjects of the aforementioned treatment, the 8 patients exhibited a significant decrease in a tumor marker level and tumor size based on CT inspection. In the 8 patients, the patients described in Example 1, 2, 5 and 6 are included.

As mentioned above, it was clearly demonstrated that administration of sodium hydrogen carbonate and vitamin C in combination and administration of them in combination with metformin are effective for treatment of cancer, particularly progressive and/or last-stage cancer. It is possible to reduce the dosage amount of an anti-cancer agent by the combined administration. A patient-friendly treatment with few side effects can be provided.

## Claims

1. A combination drug comprising a urinary alkalinization agent and vitamin C for use in treatment or remission of cancer.

2. The drug according to Claim 1, wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.

3. The drug according to Claim 2, wherein the sodium hydrogen carbonate is orally administered in a dosage of 3 to 15 g/day.

4. The drug according to any one of Claims 1 to 3, wherein the vitamin C is parenterally administered in a dosage of 25 to 50 g/day.

5. The drug according to any one of Claims 1 to 4, further comprising metformin.

6. The drug according to Claim 5, wherein the metformin is orally administered in a dosage of 250 mg to 500 mg/day.

7. The drug according to any one of Claims 1 to 6, further comprising an anti-cancer agent.

8. The drug according to any one of Claims 1 to 7, wherein the cancer is progressive cancer and/or last-stage cancer.

9. A pharmaceutical composition comprising a urinary alkalinization agent to be administered in combination with vitamin C, for use in treatment or remission of cancer.

10. A pharmaceutical composition comprising vitamin C to be administered in combination with a urinary alkalinization agent, for use in treatment or remission of cancer.

11. A pharmaceutical composition comprising metformin to be administered in combination with a urinary alkalinization agent and vitamin C, for use in treatment or remission of cancer.

12. The pharmaceutical composition according to any one of Claims 9 to 11, wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.

13. The pharmaceutical composition according to Claim 12, wherein the sodium hydrogen carbonate is orally administered in a dosage of 3 to 15 g/day.

14. The pharmaceutical composition according to any one of Claims 9 to 13, wherein the vitamin C is parenterally administered in a dosage of 25 to 50 g/day.

15. The pharmaceutical composition according to Claim 11, wherein the metformin is orally administered in a dosage of 250 mg to 500 mg/day.

16. The pharmaceutical composition according to any one of Claims 9 to 15, to be administered in combination with an anti-cancer agent.

17. The pharmaceutical composition according to any one of Claims 9 to 16, wherein the cancer is progressive cancer and/or last-stage cancer.

18. A urinary alkalinization agent for use in a method for treatment or remission of cancer together with vitamin C.

19. The urinary alkalinization agent according to Claim 18, wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.

20. The urinary alkalinization agent according to Claim 19, wherein the sodium hydrogen carbonate is orally administered in a dosage of 3 to 15 g/day.

21. The urinary alkalinization agent according to any one of Claims 18 to 20, wherein the vitamin C is parenterally administered in a dosage of 25 to 50 g/day.

22. The urinary alkalinization agent according to any one of Claims 18 to 21, for use in the method further together with an anti-cancer agent.

23. The urinary alkalinization agent according to any one of Claims 18 to 22, wherein the cancer is progressive cancer and/or last-stage cancer.

24. Vitamin C for use in a method for treatment or remission of cancer together with a urinary alkalinization agent.

25. The vitamin C according to Claim 24, to be parenterally administered in a dosage of 25 to 50 g/day.

26. The vitamin C according to Claim 24 or 25, wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.

27. The vitamin C according to Claim 26, wherein the sodium hydrogen carbonate is orally administered in a dosage of 3 to 15 g/day.

28. The vitamin C according to any one of Claims 24 to 27, for use in the method further together with an anti-cancer agent.

29. The vitamin C according to any one of Claims 24 to 28, wherein the cancer is progressive cancer and/or last-stage cancer.

30. Metformin for use in a method for treatment or remission of cancer together with a urinary alkalinization agent and vitamin C.

31. The metformin according to Claim 30, to be orally administered in a dosage of 250 mg to 500 mg/day.

32. The metformin according to Claim 30 or 31, wherein the vitamin C is parenterally administered in a dosage of 25 to 50 g/day.

33. The metformin according to any one of Claims 30 to 32, wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.

34. The metformin according to Claim 33, wherein the sodium hydrogen carbonate is orally administered in a dosage of 3 to 15 g/day.

35. The metformin according to any one of Claims 30 to 34, for use in the method further together with an anti-cancer agent.

36. The metformin according to any one of Claims 30 to 35, wherein the cancer is progressive cancer and/or last-stage cancer.

37. A method for treatment or remission of cancer, comprising administering a urinary alkalinization agent and vitamin C to a cancer patient.

38. The method according to Claim 37, wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.

39. The method according to Claim 38, wherein the sodium hydrogen carbonate is orally administered in a dosage of 3 to 15 g/day.

40. The method according to any one of Claims 37 to 39, wherein the vitamin C is parenterally administered in a dosage of 25 to 50 g/day.

41. The method according to any one of Claims 37 to 40, comprising further administering metformin.

42. The method according to Claim 41, wherein the metformin is orally administered in a dosage of 250 mg to 500 mg/day.

43. The method according to any one of Claims 37 to 42, comprising further administering an anti-cancer agent.

44. The method according to any one of Claims 37 to 43, wherein the cancer is progressive cancer and/or last-stage cancer.

45. Use of a urinary alkalinization agent and/or vitamin C in producing a medicament for use in a method for treatment or remission of cancer, comprising administering a urinary alkalinization agent and vitamin C to a cancer patient.

46. The use according to Claim 45, comprising further administering metformin in the method.

47. Use of metformin in producing a medicament for use in a method for treatment or remission of cancer, comprising administering a urinary alkalinization agent, vitamin C and metformin to a cancer patient.

48. The use according to any one of Claims 45 to 47, wherein the urinary alkalinization agent comprises one or more selected from the group consisting of sodium hydrogen carbonate, sodium carbonate, calcium carbonate, sodium citrate, potassium citrate, sodium acetate, sodium lactate, potassium lactate, magnesium oxide, magnesium hydroxide, aluminum hydroxide and aluminum silicate.

49. The use according to Claim 48, wherein the sodium hydrogen carbonate is orally administered in a dosage of 3 to 15 g/day.

50. The use according to any one of Claims 45 to 49, wherein the vitamin C is parenterally administered in a dosage of 25 to 50 g/day.

51. The use according to Claim 46 or 47, wherein the metformin is orally administered in a dosage of 250 mg to 500 mg/day.

52. The use according to any one of Claims 45 to 51, comprising further administering an anti-cancer agent in the method.

53. The use according to any one of Claims 45 to 52, wherein the cancer is progressive cancer and/or last-stage cancer.
